# EUROPEAN PATENT SPECIFICATION

(11) **EP 0 629 239 B1**
(45) Date of publication and mention of the grant of the patent: **21.07.1999**
(21) Application number: 93904013.5
(22) Date of filing: 25.02.1993
(51) Int. Cl.: C12N 15/12, C07K 14/435, A01N 63/02, C12N 7/01, C12N 15/86

(54) **INSECTICIDAL TOXINS FROM THE PARASITIC WASP BRACON HEBETOR**
Insektentötende Toxine von der parasitischen Wespe Bracon Hebetor
TOXINES INSECTICIDES DE LA GUEPE PARASITE BRACON HEBETOR

(30) Priority: 04.03.1992 US 847570; 10.06.1992 US 897192
(43) Date of publication of application: 21.12.1994
(73) Proprietor: Novartis AG, 4058 Basel (CH)
(72) Inventor: LEISY, Douglas, Jerald, Palo Alto, CA 94304 (US); QUISTAD, Gary, Bennet, Mountain View, CA 94040 (US)
(86) International application number: EP9300431
(87) International publication number: WO9318145

(56) References cited:
- CHEMICAL ABSTRACTS, vol. 86, no. 3, 17 January 1977, Columbus, Ohio, US; abstract no. 12504f, VISSER, B.J. ET AL. 'Isolation and some biochemical properties of a paralyzing toxin from the venom of the wasp Microbracon hebetor (Say)' page 88 ;
- CHEMICAL ABSTRACTS, vol. 99, no. 11, 12 September 1983, Columbus, Ohio, US; abstract no. 85452r, VISSER, B.J. ET AL. 'Characterization of two paralyzing protein toxins (A-MTX and B-MTX) isolated from a homogenate of the wasp Microbracon hebetor (Say)' page 339 ;
- VIROLOGY vol. 184, no. 2, October 1991, NEW YORK, US pages 777 - 780 MAEDA, S. ET AL. 'Insecticidal effects of an insect-specific neurotoxin expressed by a recombinant baculovirus'

## Description

This invention is directed to toxins active against insects which are isolated from the parasitic wasp Bracon hebetor, the nucleic acids which encode the toxins, cloning of the toxins, use of the toxins to control insects, and genetically engineered virus vectors carrying the toxin gene.

### BACKGROUND OF THE INVENTION

In recent years, venoms of insects and arachnids, in particular spiders and scorpions, have been investigated as a potential source of biologically active substances for use in various fields such as medicine and agriculture. Examples of such work include:
EP Patent Application, Publ. No. 208 523 A2: Glutamate Antagonists Isolated from New World Spiders Argione trifasciata and Araneus gemma.
EP Patent-Application, Publ No. 156 540: Glutamate Receptor Inibitor obtained from Nephila clavata.
Grishin et al., 1986. "Ion Channel Blocker from the Venom of Argione lobata" Biorg. Khim. 12(8):1121-1124.
Usherwood et al., 1984. "Glutamate Channel Blockade by Venoms of Argiope trifasciata and Araneus gemma" J. Physiol. Paris 79:241-245.
Aramaki et al. 1986. "Glutamate Potential Suppressor from Nephila Clavata and Nephila maculata" Proc. Japan Acad. 62, Ser B:359-362.
Usherwood et al., 1985. "Antagonism of Glutamate Receptor Channel Complexes by Spider Venom Polypeptides" Neurotoxicology 6(2):239-250.
Adams et al. 1986. "Synaptic Toxins from Agelenopsis aptera" "Insect Neurophysiology, Borkovec et al., Eds. Humana Press, Clifton, NJ 397-408.

Bracon hebetor (also known as Habrobracon hebetor and Microbracon hebetor) is a small (ca. 2 mm, less than 1 mg) ectoparasitic wasp, which has a venom that is paralytic to lepidopterans (Drenth, D. 1974, Toxicon 12:189-192). The quest to identify toxins in B. hebetor venom has continued for several years (see, e.g. Visser et al, 1976, Toxicon 14:357-307; Visser et al, 1983, Comp. Biochem, Physiol, 75B:523-530; and Spanjer et al, 1977, Toxicon 15:413-421). Most attempts have been frustrated by the lability of the toxins. Two protein toxins (mol. wt. 44 and 57 kda) have been purified and partially characterized, but they represent only 2% of the original insecticidal activity (Visser et al, supra, 1983). More recently, Slavnova et al, 1987 Doklady Akademii Nauk USSR 297:492-494 reports isolation of a toxin having a mass of 18 kda.

### DESCRIPTION OF THE INVENTION

It has now been found that certain polypeptides, when isolated from the venom of the wasp Bracon hebetor are toxic, i.e. paralytic and/or lethal to insects, particularly of the order Lepidoptera, at surprisingly low concentrations.

The present invention, therefore, concerns toxins free from associated wasp polypeptides which demonstrate toxicity towards insects. These polypeptides may be isolated from, or be constructed to show substantial sequence homology to polypeptides isolated from the venom of Bracon hebetor. Preferred peptides are rather large, and may be characterized in having a molecular weight which exceeds 70,000 da and comprise the sequence of SEQ.ID. NO.1, SEQ.ID. NO.2, SEQ.ID. NO.3 or SEQ.ID. NO.5. Five polypeptides were isolated and were designated Brh-I to Brh-V.

As used throughout the specification and claims, the following definitions are intended: Associated wasp polypeptides-- polypeptides naturally occurring in the venom of B. hebetor which are toxic to insects.

Homologous polypeptide-- a polypeptide which is identical to one of the native toxins of this invention, or substantially homologous (at least 80%) with respect to the amino acid sequence, such that it demonstrates substantially the same insect toxicity in in vivo assays as a native toxin.

Homologous nucleotide sequence-- a sequence which will hybridize to the reference sequence under stringent hybridization conditions.

Stringent hybridization conditions-- those in which hybridization is effected in a standard manner at 65°C in 4X buffered saline (a.k.a. SSPE buffer) followed by merely washing at 52°C in 0.2 X SSPE, which will not affect true hybrids which have formed.

### DESCRIPTION OF THE FIGURES

Figure 1 demonstrates the purification of toxins from 300 B. hebetor venom glands using anion-exchange chromatography.

Figure 2 is the reversed-phase HPLC analysis of combined fractions 7-13 from Figure 1, using 25 gland-equivalents.

Figure 3 is the reversed-phase HPLC analysis of Fraction 8 from Figure 1 (25 gland equivalents). This is predominantly Brh-I.

Figure 4 is the reversed-phase HPLC analysis of Fraction 11 from Figure 1 (25 gland equivalents). This is predominantly Brh-V.

Figure 5 is the size-exclusion HPLC analysis of toxins from 80 B. hebetor venom glands.

Figure 6 is a diagram of plasmid pSCI810, containing the Brh-I gene.

Figure 7 is a diagram of plasmid pSCI277, containing the Brh-I gene linked to the p10 promoter.

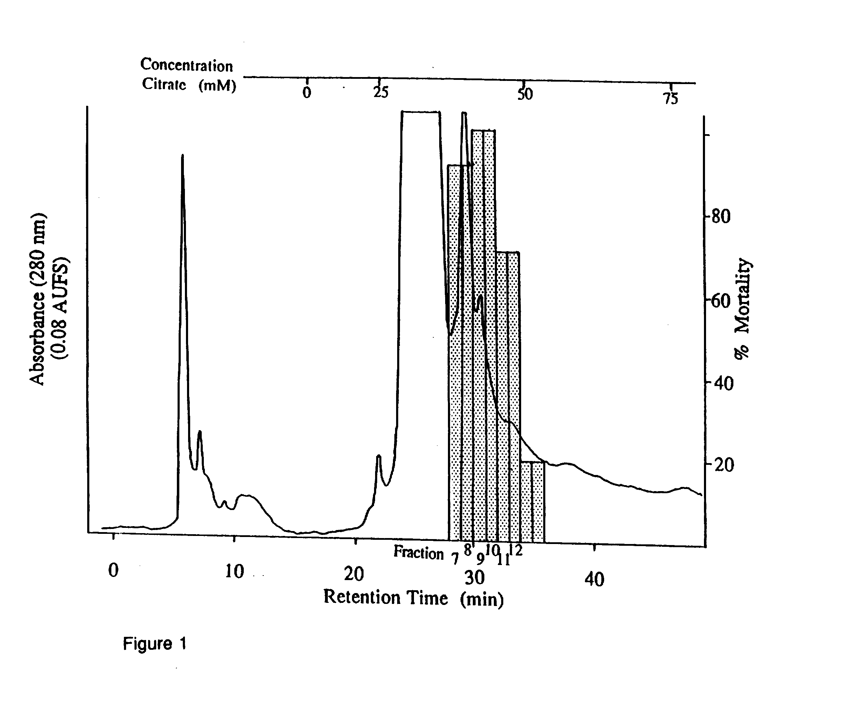

The toxins of this invention are quite labile under many isolation conditions. As they are particularly unstable at low pH, reverse-phase HPLC was contraindicated. The successful structure elucidation of toxins which are part of this invention is predicated on the purification by anion-exchange chromatography which was monitored by reversed-phase HPLC. The results of the anion-exchange chromatography are shown in Figure 1. Various fractions, designated Fractions 7-13 are identified, and their insect mortality is assessed.

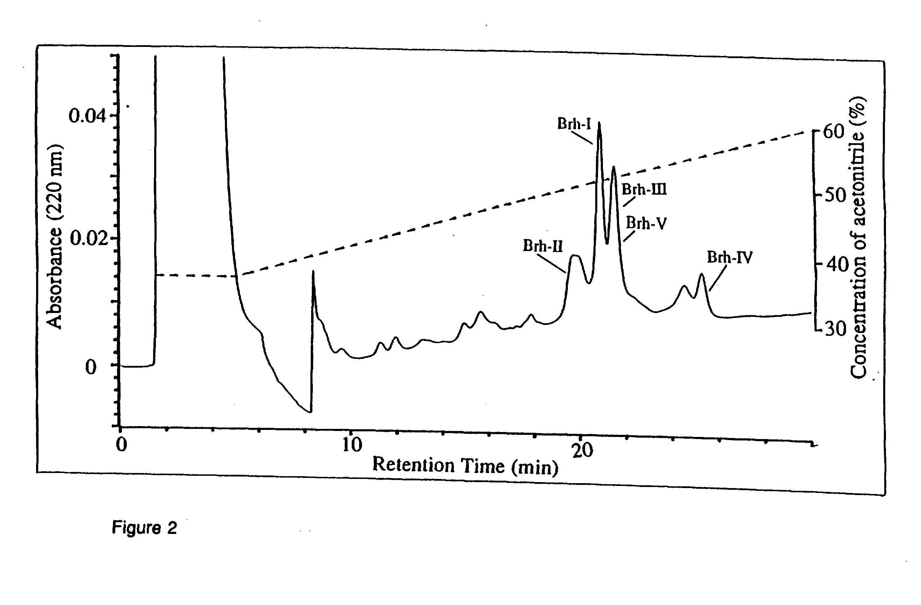

As ultraviolet absorbance was of limited utility during toxin purification by ion-exchange, short (1 min) fractions were collected relative to contaminant absorbance (280 nm) and each fraction was assayed for bioactivity and purity (typically 25 gland equivalents using reverse-phase HPLC). Results are illustrated in Figures 2, 3, and 4.

In order to consistently recover toxins exhibiting high bioactivity, it was necessary to add a protein carrier (bovine serum albumin) prior to desalting by membrane filtration. Purified toxins could not be freeze-dried, even in the presence of BSA and were best stored in a solution (0.02 M ammonium carbonate, pH 9.2, 4°C). Under these conditions, the toxins were stable for at least two weeks.

Thus one aspect of this invention is a polypeptide free from associated wasp polypeptides substantially homologous to a polypeptide isolated from Bracon hebetor and exhibiting insect toxicity. Preferred polypeptides have a molecular weight of at least 70,000 da. Examples of prefered polypeptides of this invention include: Brh-I, Brh-II, Brh-III, Brh-IV and Brh-V.

The preferred polypeptides of this invention may be characterized by their behavior during Anion-Exchange and Reversed-Phase HPLC as follows, the complete conditions of which are given in Example 1. By following the procedures set forth in Example 1, ie. Anion-Exchange chromatography followed by Reversed-Phase chromatography, peaks characteristic of Brh-I, Brh-II, Brh-III, Brh-IV, and Brh-V can be identified.

Three of the five native toxins were sequenced at least partially. Results are presented below.

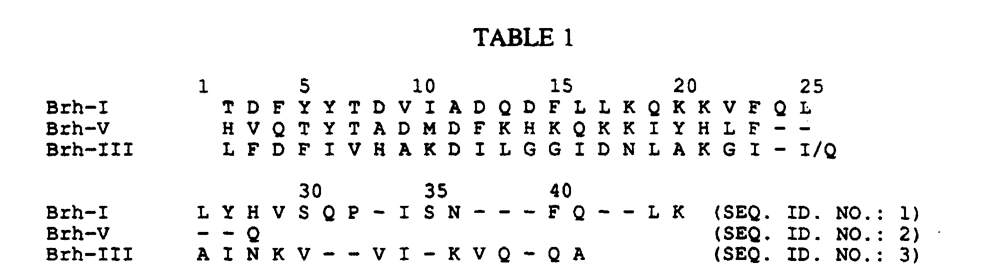

The polypeptides of this invention may be prepared by a variety of techniques. They may, for example, be isolated from the crude venom of B. hebetor using purification techniques, such as those presented in the Examples. Alternatively, with knowledge of the amino acid sequence of the polypeptides, synthetic construction, using conventional protein synthesis techniques may be employed.

A further technique which may advantageously employed in the production of polypeptides of this invention involves the construction, by conventional methods, of a DNA sequence which, upon expression, encodes a polypeptide according to this invention. Such DNA sequences may then be inserted into an appropriate vector, either alone or in combination with other homologous or heterologous DNA sequences whose function may be to control the expression of the polypeptide-encoding DNA sequence of interest or may result in, for example, a fusion protein, enhancing or extending the activity of the toxin DNA expression product therefrom. Suitably employed as vectors are plasmids, phages, and viruses, the use of which for such purpose is common knowledge to the ordinary artisan. Cells in which a vector containing such a toxin DNA may be expressed, include, for example, prokaryotic cells such as E. coli and Bacillus spp., or eukaryotic cells such as yeast cells or insect cells.

A preferred method for producing the toxin polypeptides directly as a toxic product such that no work-up towards isolation, purification, and formulation of an expression product is required is by employing an insect specific virus (baculovirus) as a vector. A gene encoding the desired polypeptide toxin is inserted into the baculovirus DNA, and is under the control of a baculovirus promoter. After the recombinant hybrid baculovirus is ingested by the insect, the virus multiplies inside the insect and the toxin is expressed (produced) in an amount sufficient to enhance the virus' insecticidal effect on the insect. Such a recombinantly modified baculovirus DNA may also be used as a vector for the introduction of the wasp toxin-producing gene into cells, particularly insect cells, to provide further systems for the production of toxins.

Therefore, also provided is a method of controlling insects comprising infecting the insects with a recombinant hybrid baculovirus.

A number of baculoviruses suitable for use as vectors, and are known in the art, such as the nuclear polyhedrosis virus from Autographa californica, Heliothis virescens, and Bombyx mori. Suitable techniques are described, for example in European Patent Application 0175 852 and U.S. Patent 4,745,051, both of which are hereby incorporated by reference.

Thus, other aspects of this invention are nucleic acids sequences (RNAs and DNAs) comprising those which encode toxin polypeptides and nucleic acid sequences which are substantially homologous to a native sequence. The nucleic acid sequences of this invention may also include sequences which are not expressed in the final polypeptide product, such as signal sequences, termination sequences, and the like.

A further aspect of this invention, therefore involves the cloning and genetic engineering of genes encoding the various toxins, and in particular Brh-I. While Brh-I is presented as an Example, any of the polypeptides of this invention may be similarly sequenced and cloned

Starting with approximately 3.1 g of wasps, approximately 8 µg of poly A+ mRNA was obtained using the procedures detailed in the Examples. Degenerate oligonucleotide primers corresponding to two regions of the nucleotide sequence obtained by reverse translation of the mature Brh-I peptide were synthesized and used for PCR amplification from B. hebetor mRNA. DNA fragments with the expected size of approximately 130 bp were produced in the PCR reaction. The DNA fragments were gel purified, cloned into pTZ18R, and three clones were sequenced. All three of these clones contained a reading frame that matched a portion of the amino acid sequence of mature Brh-I toxin. A nondegenerate primer designed to match a region from within the amplified sequence was end-labelled with ³²P and used to screen a λZAPII cDNA library made from B.hebetor. Three positive plaques were detected in a library screening of approximately 1.2 X 10⁶ plaques.

After plaque purification and in vivo excision of the cDNA containing pBluescript SK-plasmids from the λZAPII clones, the cDNA inserts of 3 clones were subjected to DNA sequence analysis. In order to determine the expected size of a full length cDNA, a primer extension reaction was performed with B. hebetor mRNA. The nucleic acid and amino acid sequence of Brh-I is given in TABLE 2 (SEQ. ID. NO.: 4 and SEQ. ID. NO.: 5). Translational initiation very likely occurs at the ATG as indicated in TABLE 2 because a) this is the first methionine codon encountered in the cDNA; b) the codon for methionine is found in the sequence ATAATGC, which conforms with the ribosome initiation site consensus sequence determined by Kozak, M., 1989, J. Cell Biol. 108:229-241; and c) there is a translational stop sequence, TAA, in frame with the Brh-I open reading frame just upstream from this methionine codon. The predicted translation product for the cDNA is a molecule of 678 amino acids. The amino acid sequence given in TABLE 2 has an 18 amino acid sequence at the N-terminus preceding the sequence determined by analysis of the isolated polypeptide (Table 1). As this 18 amino acid sequence has many of the properties expected for a signal sequence (see, e.g., von Heijne, G. Nucl. Acids Res. 14:4683-4690) it appears that this sequence is a signal sequence which is cleaved after translation.

The molecular weight of mature Brh-I predicted from the cDNA sequence is 77,912. This is somewhat larger than the value determined by SDS-PAGE analysis of isolated Brh-I (approximately 73,000), however, the amino acid composition determined for Brh-I and for the cDNA translation product agree within experimental error, suggesting that no other extensive proteolytic processing occurs besides signal sequence cleavage.

Because of the very high level of paralytic activity that Brh-I elicits upon injection of a member of different insects, cDNAs encoding Brh-I toxin may be cloned in an insect baculovirus. Upon expression in the insect, there will be a quicker cessation of feeding than occurs after infection with wild type baculoviruses. Insect baculoviruses occur in two forms, occluded viruses, which are responsible for the spread of viruses between insects, and nonoccluded or budded viruses which are responsible for the cell to cell spread of viruses within an infected insect. Infection of insects per os normally requires the occluded form of the virus. Thus a further aspect of this invention is a recombinant virus containing a gene encoding a toxin of this invention inserted at a locus such that occlusion body formation is not disrupted. One such locus is the p10 locus.

Polypeptides isolated from or those showing substantial homology to those isolated from the venom of B. hebetor are useful as insect toxic agents. In particular, they are useful toxic agents against insects of the order Lepidoptera, for example, Heliothis virescens, Autographa californica, and the insects of the genus Spodoptera. Both the purified toxin and viruses transformed to produce the toxin are assayed for bioactivity on larvae including: tobacco hornworms (Manduca sexta), tobacco budworms (Heliothis virescens) and beet armyworm (Spodoptera exigua). Toxicity is demonstrated by the ability of the polypeptides to cause paralysis and/or death of the test larvae.

The present invention also provides the use of polypeptides isolated from, or polymptides showing substantial sequence homology to those isolated from Bracon hebetor as insect toxic agents. Thus a further aspect of the invention is a method of controlling insects comprising exposing the insects to an insect controlling amount of a polypeptide of the invention. For use as insecticides, the recombinant viruses which produce polypeptides of the invention may be combined with suitable carrier substances such as those typically found in insect control formulations, such as adjuvants, diluents, modifiers or conditioning agents. The formulations may be in the form of solutions, emulsions, dispersions, powders, dusts, granules and the like. It may be advantageous to include a surface active agent such as DMSO in the formulation so that the toxin passes directly through the cuticle of the insect and avoids the digestive enzymes which might affect its activity.

These compositions are advantageously applied to the insect or its locale in an amount suitable to control the target insects. By control, as used herein, is meant the induction of paralysis, mortality, or cessation of eating. Dosages of the composition of the invention will depend on numerous factors, including the pest to be controlled and the climatic conditions, but will generally be in the range of 0.5 to 100 kg/hectare, preferably 10-50 kg/hectare.

The following non-limiting Examples are presented to better illustrate the invention.

### EXAMPLE 1

### Isolation of Toxins

B. hebetor adult wasps are purchased from Biofac, Inc., Mathis TX. Upon arrival, wasps are frozen at -20°C until used. Venom glands and associated tissue are removed from female wasps and are stored in 1.5 ml polypropylene tubes at -20°C until processed.

Purification. Lots of 100 venom glands are homogenized manually with a glass pestle in 1 ml water. After centrifugation at 3000 x g, the supernatants from 300 glands are passed through Bio-Rad 10 DG size-exclusion columns. The excluded fraction (mol. wt >6 kda) is purified by ion-exchange chromatography.

Anion-exchange chromatography is performed with a Perkin-Elmer pump (410 BIO), a Spectra-Physics ultraviolet detector (Model 8300, 280 nm) and an Altex column (Spherogel-TSK, DEAE-5PW, 7.5 X 75 mm) with a elution at 1 ml/min. The elution solvent is citrate in 0.02 M ammonium carbonate containing 10% aceto-nitrile, pH 8.2: 0 mM citrate for 15 min, linear gradient to 25 mM over 5 min, and 25 to 75 mM over 50 min. Individual fractions are bioassayed using M. sexta larvae. As shown in Figure 1, all insecticidal activity is found in one broad zone (Fractions 7-13). Collectively, these fractions contain several wasp toxins (See Fig. 2, 3 and 4), but only 59 pg total protein from 300 venom glands, representing an approximately 4000-fold purification of toxins based on initial whole wasp mass.

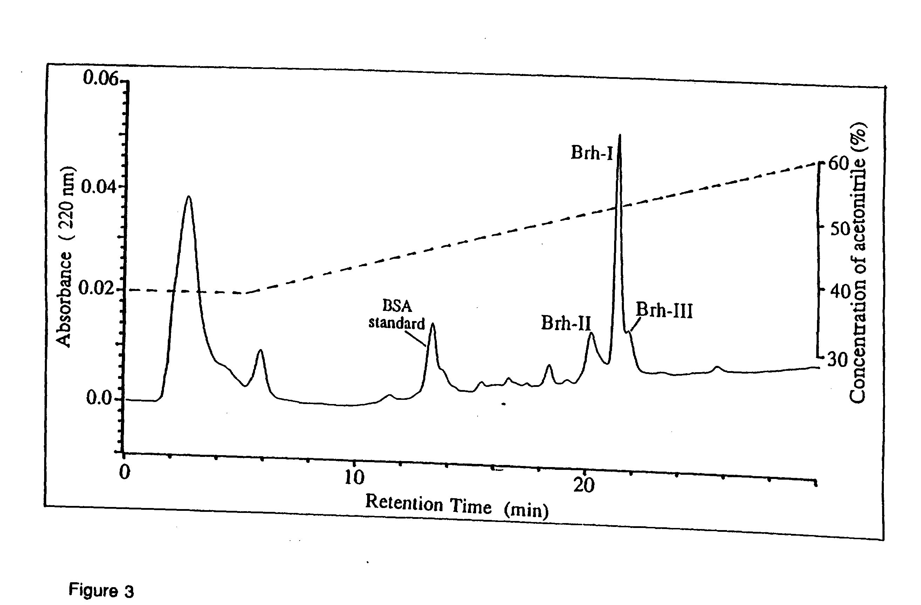

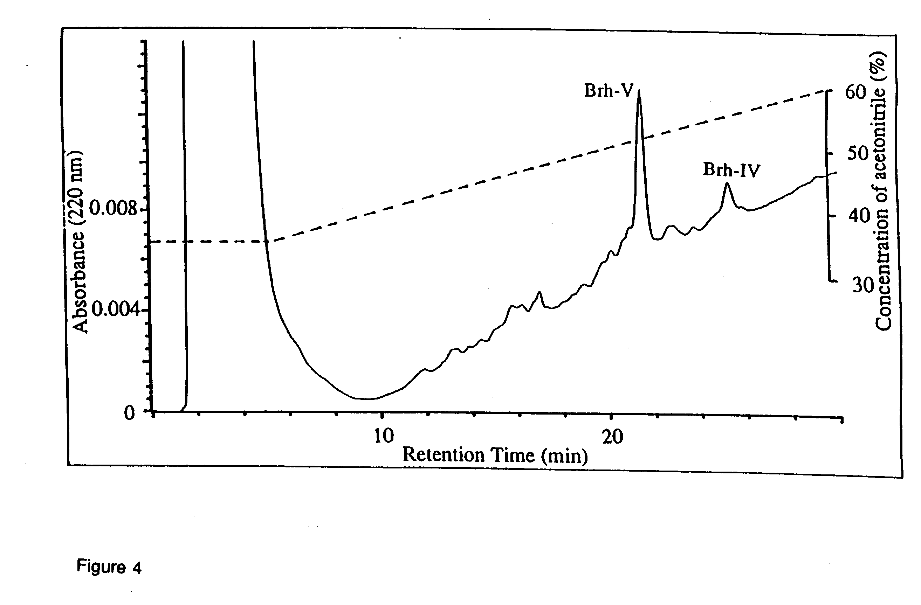

Reverse phase liquid chromatography (HPLC) is performed using a Hewett Packard (HP) pump (Model 1090), an HP diode array detector (Model 1040, 220 and 280 nm), and a narrow-bore Vydac C₄-300 A column (15 X 0.2 cm) with a flow rate of 0.3 ml/min. The eluent is acetonitrile in 0.1% trifluoroacetic acid: 35% for 5 min, linear gradient 35 to 60% over 25 min. HPLC demonstrates that most individual fractions from ion-exchange chromatography are mixtures of toxins. However, fractions 8 and 11 are sufficiently pure to allow further characterizations. Fraction 8 contains predominantly Brh-I while Fraction 11 contains mostly Brh-V (See Figs. 3 and 4).

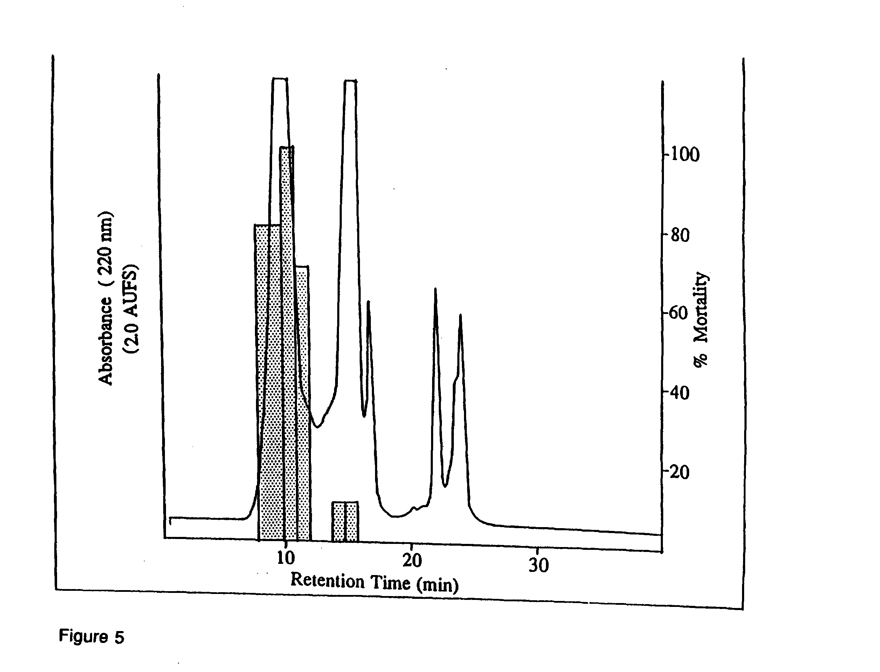

Size-exclusion chromatography employed the same Perkin-Elmer pump and Spectra-Physics detector as above with an Altex Spherogel-TSK 3000SW column (7.5 x 300 mm) with a flow of 0.5 ml/min. The eluent is 10% acetonitrile. Results are shown in Figure 5.

### EXAMPLE 2

### BIOASSAY

The bioactivity of whole venom glands is determined for 50 glands homogenized in ice water. After centrifugation at 3000 x g, 3 pl aliquots are injected into the pronotum of lepidopteran larvae which are scored for mortality at 24 hrs. The lethal dose for 50% of the treated larvae (LD₅₀) is calculated by Probit analysis from duplicate lots of glands, each of with at least four dose rates.

Purified Brh-I and Brh-V toxins are assayed similarly. Prior to the bioassay, 1 min. fraction from the ion-exchange purification are desalted using Centricon®-10 microconcentrators (Amicon). Bovine serum albumin (100 µg) is added to samples prior to desalting to improve recovery of toxins. Toxins are concentrated to approximately 150 µl in 0.02 M ammonium carbonate for bioassay.

The following larvae are bioassayed: Manduca sexta (tobacco hornworm) third stadium; Heliothis virescens (tobacco budworm) fourth stadium; Helicoverpa zea (corn earworm) fourth stadium; Spodoptera exigua (beet armyworm) fifth stadium; Galleria mellonella (wax moth) fourth stadium; TrichoDlusia ni (cabbage looper) fifth stadium; Pieris rapae (cabbage butterfly) third stadium; and Diabrotica undecimpunctata (western spotted cucumber beetle) third stadium. Results are presented in Tables 3 and 4, below.

**TABLE 3**

| Bioactivity of Venom Gland Extract | | | |
|---|---|---|---|
| | mass of larva (mg) | LD₅₀ glands/larva | LD₅₀ glands/g |
| M. sexta | 47 | 0.036 | 0.77 |
| H. virescens | 48 | 0.080 | 1.7 |
| S. exigua | 50 | 0.065 | 1.3 |
| G. mellonella | 61 | 0.00046 | 0.0076 |
| H. zea | 41 | 0.038 | 0.95 |
| T. ni | 66 | 0.0014 | 0.021 |
| D. undecimpunctata | 13 | >0.2 | >13 |
| P. rapae | 56 | 0.000073 | 0.0013 |

**TABLE 4**

| Injection Assay | | |
|---|---|---|
| | LD₅₀ (µg/g) Brh-I | LD₅₀ (µg/g) Brh-V |
| M. sexta | 0.05* | 0.04* |
| S. exigua | 0.033* | 0.051* |
| H. virescens | 0.18* | 0.26* |
| H. zea | 0.045* | 0.085 |
| G. mellonella | 0.0023* | 0.00011 |
| T. ni | 0.019 | 0.0038 |

| | | |
|---|---|---|
| * Duplicate toxin isolation for bioassay | | |

### EXAMPLE 3

### Sequence Analysis

900 venom glands are processed as described above. Purified toxins (43-77 pmol) are sequenced twice using an Applied Biosystems Model 477A pulsed liquid phase protein sequencer. Released phenylthiohydantoin amino acids are analyzed using an on-line analyzer (Applied Biosystems Model 120A). Brh-I is also converted to a reduced, carboxymethylated derivative prior to sequencing as described in Skinner et al, 1989 J. Biol. Chem, 264:2150-2155, which is hereby incorporated by reference.

Toxins Brh-I and Brh-V are analyzed for constituent amino acids after hydrolysis in vacuo by vapor from 6 M HCl/1% phenol at 110°C for 20 hrs. Hydrolysates are analyzed using a Hewlett Packard AminoQuant amino acid analyzer.

### EXAMPLE 4

### Isolation of mRNA

B. hebetor wasps are stored at -80°C. 3.15 g of both male and female wasps are homogenized with a Polytron for 1 min in 20 ml RNA extraction buffer (4 M guanidine isothiocyanate, 50 mM sodium citrate, pH 7.0, and 0.1 M 2-mercaptoethanol). Following homogenization, 1 ml 15% Sarkosyl is added. The homogenate is centrifuged at 8,000 rpm for 10 min at 4°C in a Sorvall HB-4 rotor, and the supernatants are decanted into clean tubes to remove insoluble debris. This is repeated once and the supernatant is layered over 3 ml of 5.7 M CsCl in 0.01M EDTA, pH 8.0, and is centrifuged for 17 hours at 35,000 rpm in a Beckman Ti55 rotor. The pellet is resuspended in 15 ml of FastTrack Lysis Buffer (Invitrogen Corp) and the mRNA is then isolated following the protocol provided by Invitrogen Corp for their FastTrack mRNA isolation kit.

### EXAMPLE 5

### PCR Amplification

Single-stranded cDNA is synthesized from B. hebetor mRNA (0.5 µg) using M-MLV reverse transcriptase (GIBCO-Bethesda Research Laboratories) primed with a degenerate 20-mer oligonucleotide primer with the following sequence (SEQ. ID. NO.: 6) This primer is complementary to a sequence derived by reverse translation of the Brh-I toxin N-terminal amino acid sequence. Following the cDNA synthesis, the reactions are heated to 90°C for 5 minutes, cooled to room temperature and ethanol precipitated. The cDNA reaction product is amplified in a 50 µl reaction with PCR geneAMP reagents (Perkin-Elmer Cetus Instruments), using 2 µM each of the above primer and a second degenerative primer with the following sequence (SEQ. ID. NO.: 7): This primer corresponds to a second portion of the reverse translation product of the Brh-I toxin N-terminal amino acid sequence. PCR conditions are as follows: 1 min at 94°C; 2 min at 37°C; slow ramping of the temperature over 3 min to 72°C; 3 min at 72°C; 10 sec extension of the 72°C segment per cycle for 30 cycles; and a final cycle extension of 72°C segment for 10 min. The products of the PCR reaction are electrophoresed on a 2% Agarose gel in TBE buffer (Sambrook et al, 1989 Molecular Cloning; A Laboratory Manual, 2nd Ed, Cold Spring Harbor Laboratory Press, Cold Spring Harbor, NY). A gel slice expected to contain the approximately 70 bp product is removed, and the DNA is isolated using Geneclean (Bio101) reagents according to the manufacturer's instructions. The DNA is then reamplified using the same primers and the same PCR conditions as described above.

The DNA fragments are then gel-purified and cloned into pTZ18R (BIO-RAD Laboratories) and three clones are sequenced All three clones match the Brh-I sequence from Table 1. A non-degenerate primer, designed to match a region from within the amplified sequence (See Table 5) is end-labeled with ³²P and used to screen a λZAPII cDNA library made from B. hebetor mRNA. Three positive plaques are detected in a library screening of approximately 1.2 X 10⁶ plaques. One of the Brh-I positive clones has an insert size of approximately 3.0 kb, and the other two each have inserts of approximately 2.3 kb.

### TABLE 5

### Reverse translation and PCR Amplification of Brh-I toxin mRNA.

The amino acid sequence of amino acids 2 through 32 from the N-terminus of Brh-I toxin is shown on line 1. The nucleotide sequence derived by reverse translation of the Brh-I amino acid sequence is shown below the amino acid sequence on line 2. All possible nucleotides at each position are indicated; Y= C or T; R= A or G; M= A or C; W= A or T; H= A, C, or T; N= A, C, T, or G. Degenerate oligonucleotides corresponding to the first underlined region and the complement of the second underlined region are used as PCR primers for amplification from Brh-I mRNA. Lines, 3, 4, and 5 show the nucleotide sequence from free cloned PCR fragments. Underlined sequences correspond to primer regions. Line 6 shows the sequence of the nondegenerate oligonucleotide which was used as a hybridization probe for screening the B. hebetor cDNA library.

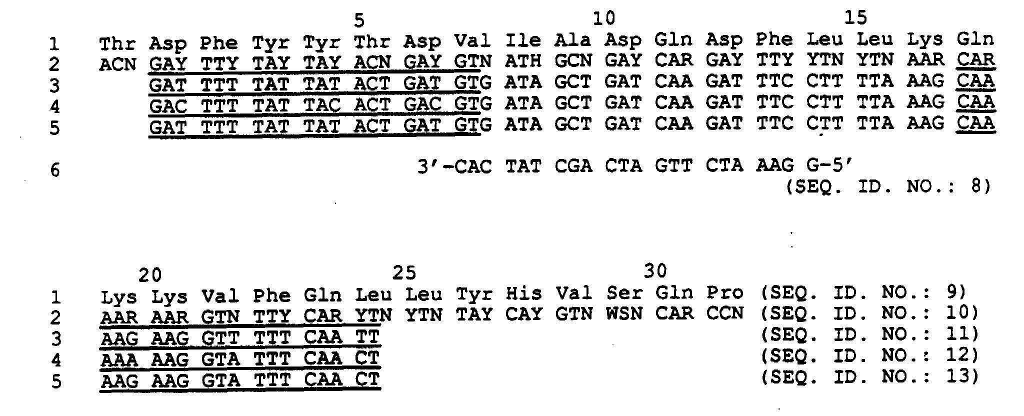

DNA sequencing and Sequence Analysis. Double stranded pBluescript SK- plasmid DNA containing cDNA inserts are generated from λZAPII cDNA clones following the in vivo excision procedure described in the λZAPII instruction manual. The 5'-termini of the cDNA inserts are then subjected to PCR amplification using the primer indicated in Table 5 coupled with M13 reverse primer. The PCR products from the 5'-termini of the 2.3 kb cDNA inserts have identical sequences and that from the 3.0 kb cDNA sequence is the same as those of the 2.3 kb cDNA except for being shorter by four nucleotides.

The two XhoI fragments from within the cDNA insert are subcloned into the pBluescriptSK+II vector, and unidirectional deletion series are generated from both ends of each clone. Both strands of the cDNA are sequenced in their entirety, and is given in Table 2 (SEQ. ID. NO. 4 and SEQ. ID. NO.: 5).

The nucleotide sequences and predicted protein sequences are analyzed with the IntelliGenetics suite of sequence analysis programs.

### EXAMPLE 5

### Construction of Vectors

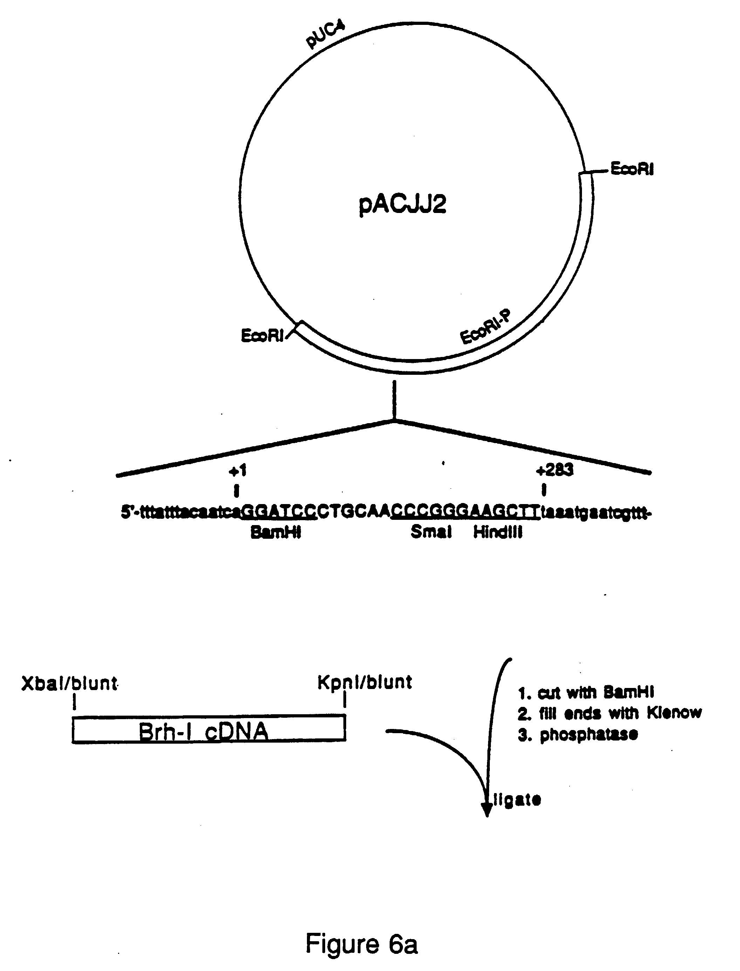

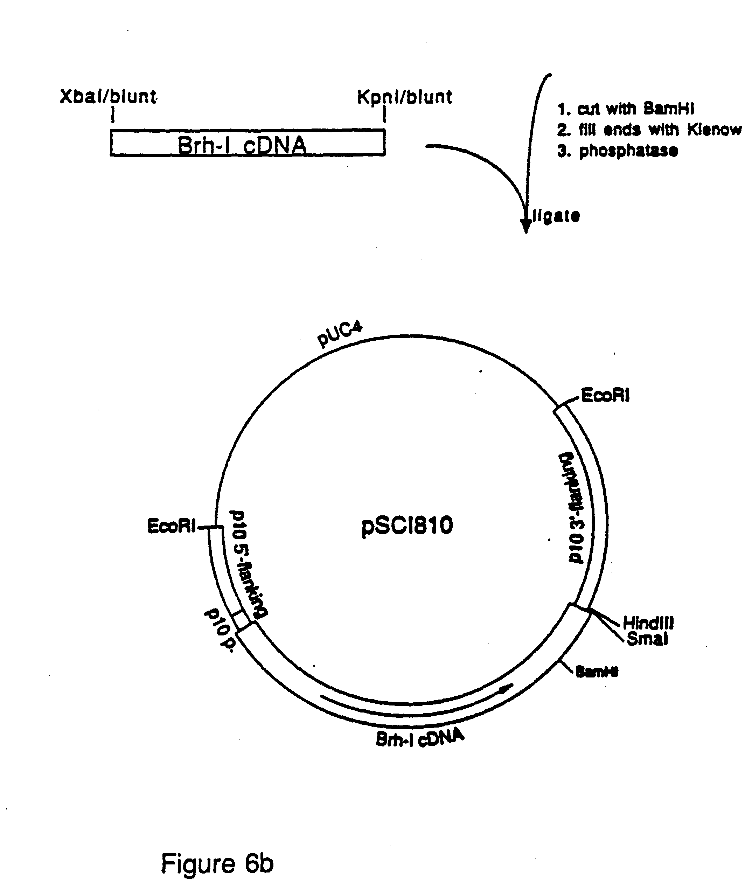

A plasmid containing the Brh-I cDNA is liberated from a recombinant λZAPII isolate by in vivo excision. The plasmid is cut with XbaI and KpnI, treated with Klenow in the presence of all four dNPTs to make the ends blunt, and then the cDNA containing insert is gel isolated. The plasmid pACJJ2, (obtained from Dr. Just Vlak, Dept. of Virology, Agricultural Univ., Wageningen, The Netherlands) which has a polylinker in place of the complete p10 open reading frame, is cut with BamHI, the ends are filled, phosphatased with calf intestinal phosphatase, and then is ligated with the isolated Brh-I cDNA to form pSC1810, as shown in Figure 6.

Next, pBluescript KS⁺ and pBluescript SK⁺ are each digested with KpnI and XholI, treated with Klenow in the presence of dNPT's to make the ends flush, and then religated to form pBluescript KS⁺ΔXK and pBluescript SK⁺ΔXK respectively. Each of these plasmids is then digested with ScaI and Sail. The larger of the two fragments from the Scal and SalI digested pBluescript KS⁺ΔXK and the smaller of the two fragments from the Scal and SalI digested pBluescript SK⁺ΔXK are isolated and ligated together to form pSCI235. Another plasmid, pSCI839 is constructed by subcloning an approximately 3 kb SalI fragment containing an intact polyhedrin gene into pSCI234 to form pSCI839. The SalI fragment containing the polyhedrin gene in pSCI839 is isolated from pSCI275, which is a pWE15-based cosmid vector containing a large segment of AcNPV DNA (randomly cloned from AcNPV strain LI) that includes the polyhedrin gene.

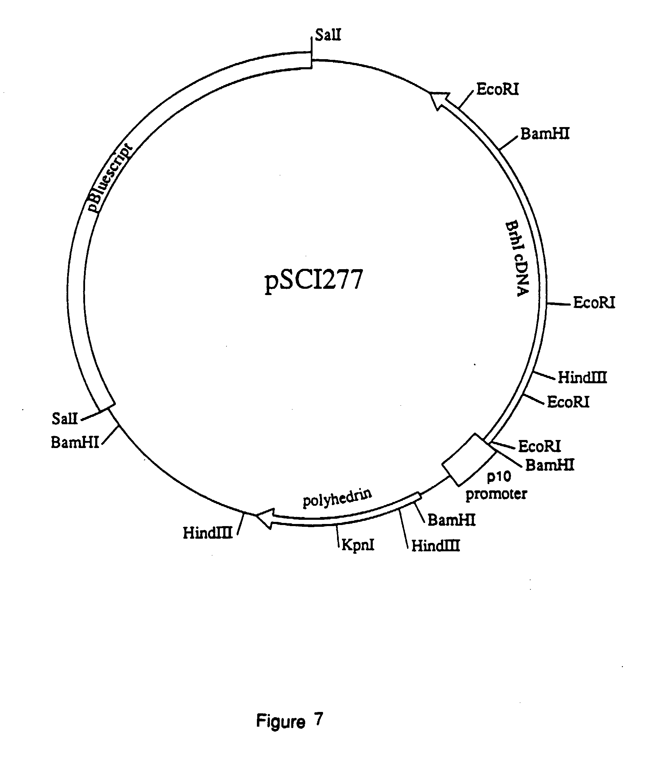

Plasmid pSCI276 is formed by cutting pSCI810 with PstI and SpeI, making the ends blunt with T₄ DNA polymerase in the presence of dNTPs, and is religated. pSCI276 is then cut with SphI and SmaI, the SphI ends are made flush with T₄ DNA polymerase in the presence of dNTPs, and an approximately 2650 bp segment containing the Brh-I cDNA linked to the p10 promoter is isolated and cloned into the unique EcoRV site of pSCI839 to form pSCI277. The plasmid pSCI277 has a module containing the p10 promoter region linked to the Brh-I cDNA inserted at the EcoRV site just upstream of the intact polyhedrin gene; it is diagrammed in Figure 7. After transfection of this plasmid with polyhedrin⁻ viral plasmid DNA, recombinant viruses are selected by their ability to form polyhedra⁺ plaques.

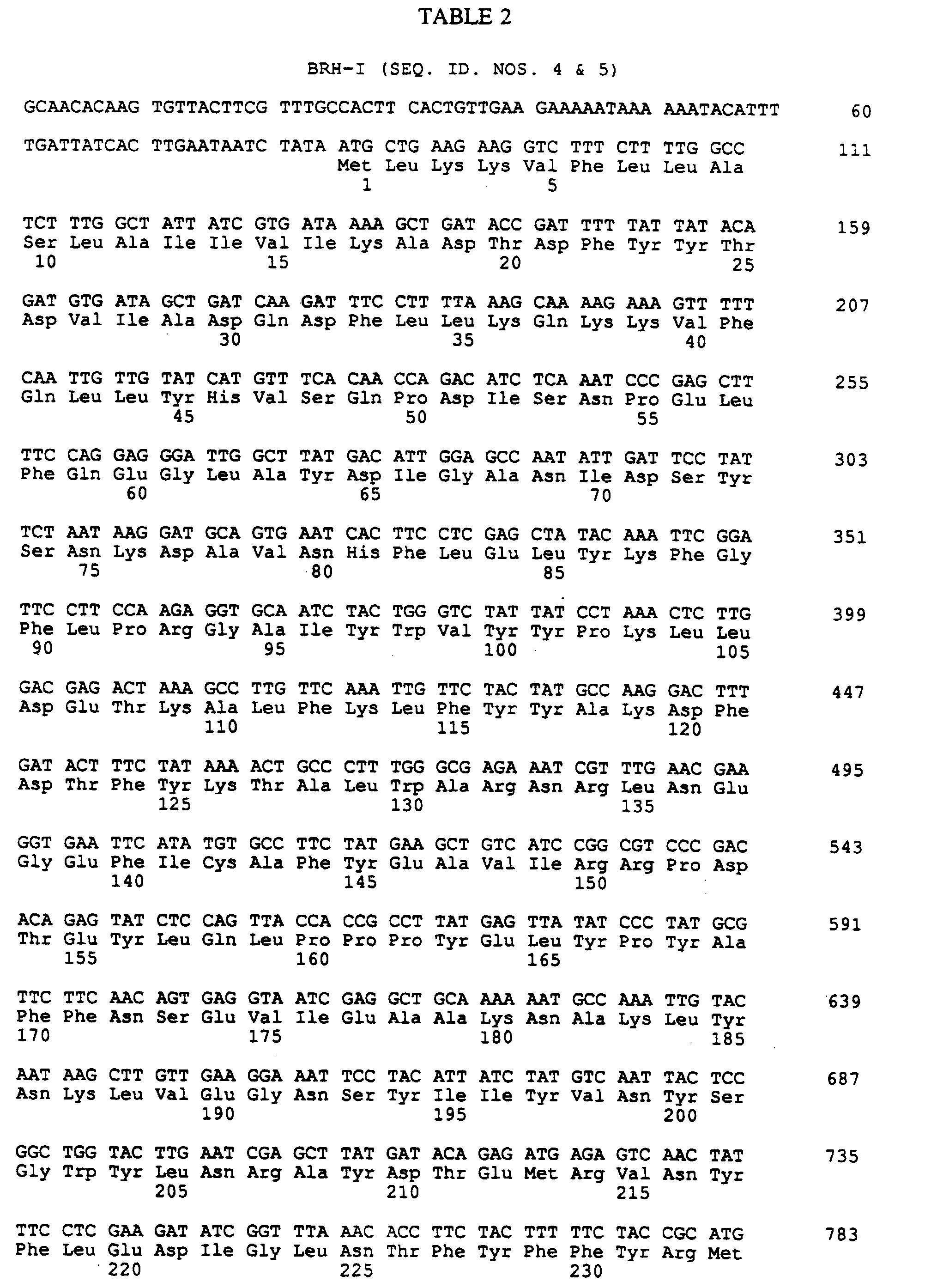

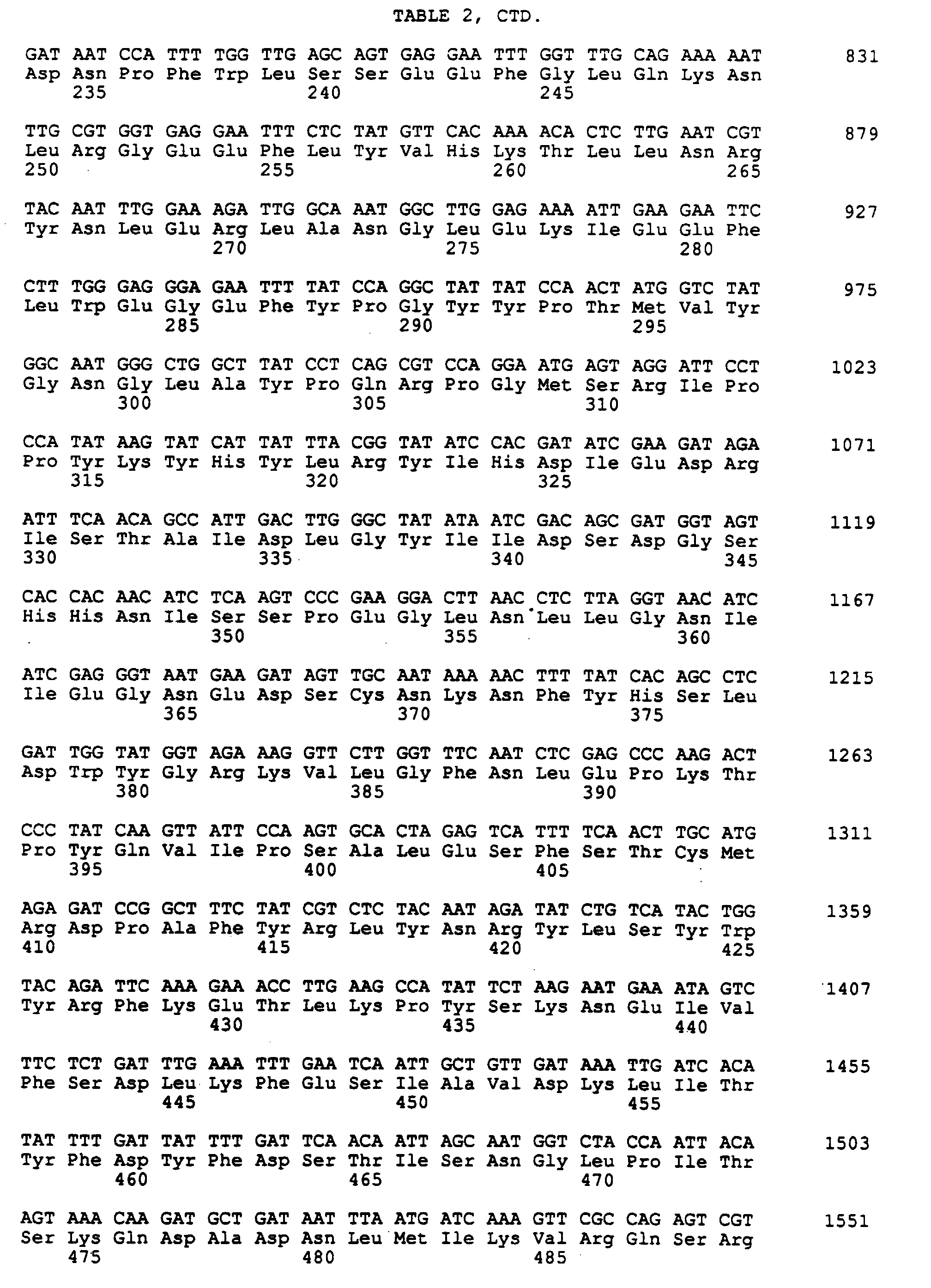

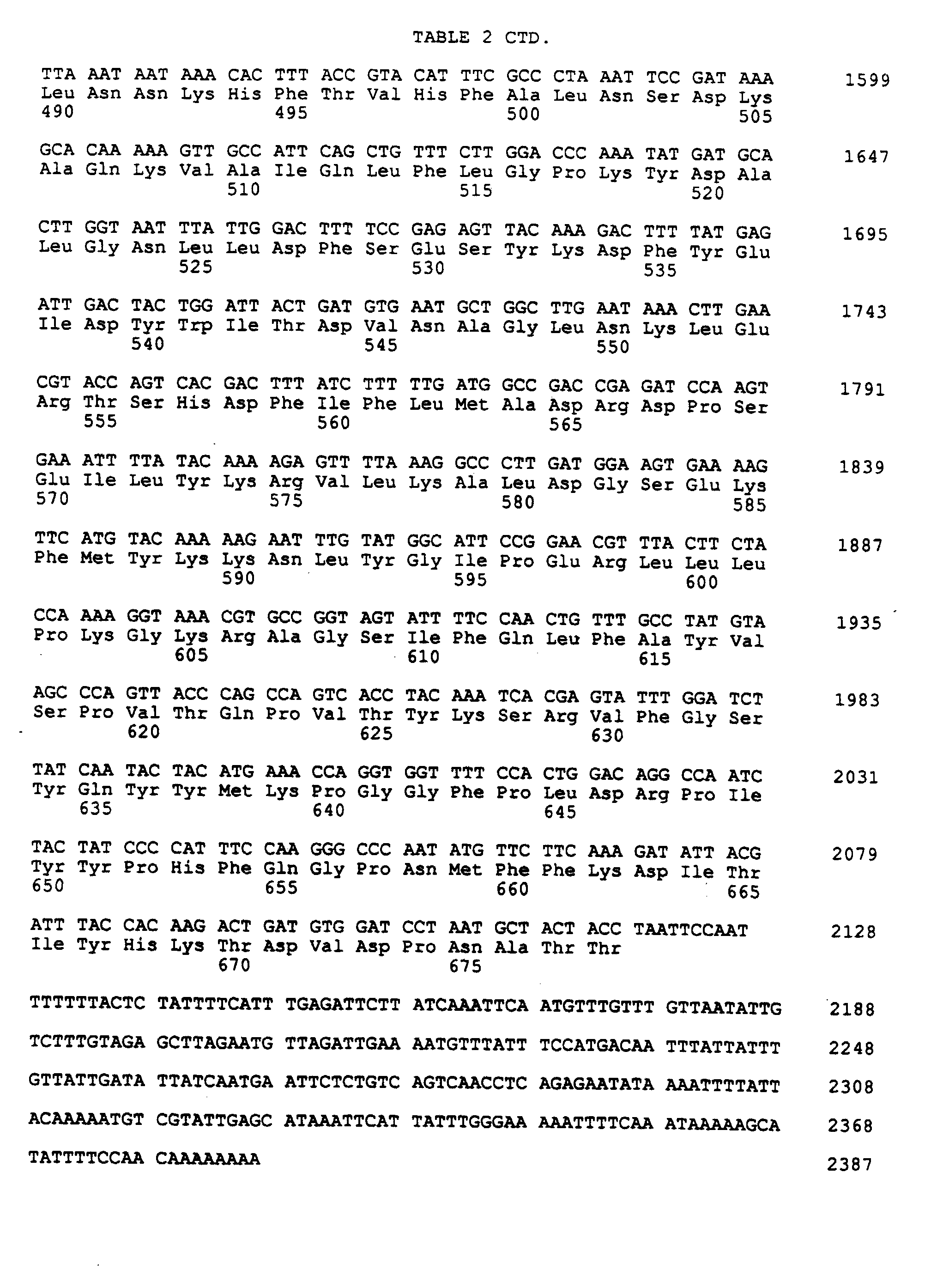

## Claims

1. A polypeptide isolated from the wasp *Bracon hebetor* which exhibits insect toxicity, has a molecular weight greater than 70 kDa and comprises the sequence of SEQ ID No. 1, SEQ ID No. 2 or SEQ ID No. 3.

2. A polypeptide which exhibits insect toxicity, has a molecular weight greater than 70kDa and is at least 80% homologous to one of the polypeptides defined in Claim 1.

3. A polypeptide designated Brh-1 isolated from the wasp *Bracon hebetor* which exhibits insect toxicity, has a molecular weight of greater than 70kDa and has the sequence of SEQ ID No. 5.

4. A polypeptide which exhibits insect toxicity, has a molecular weight greater than 70kDa and is at least 80% homologous to the polypeptide defined in Claim 3.

5. A nucleic acid encoding a polypeptide according to any one of Claims 1 to 4.

6. A nucleic acid according to Claim 5 which is DNA.

7. A vector comprising the nucleic acid of Claim 5.

8. A baculovirus whose genome comprises a nucleic acid of Claim 5.

9. A method of controlling insects comprising exposing the insects to an insect controlling amount of a polypeptide according to any one of Claims 1 to 4.

10. A method of controlling insects comprising infecting the insects with a baculovirus defined in Claim 8.

## Patentansprüche

1. Polypeptid, isoliert aus der Wespe *Bracon hebetor,* das Insektentoxizität zeigt, ein Molekulargewicht von mehr als 70 kDa besitzt und die Sequenz von SEQ ID No. 1, SEQ ID No. 2 oder SEQ ID No. 3 umfaßt.

2. Polypeptid, das Insektentoxizität zeigt, ein Molekulargewicht von mehr als 70 kDA besitzt und zu mindestens 80% homolog zu einem der in Anspruch 1 definierten Polypeptide ist.

3. Polypeptid mit der Bezeichnung Brh-1, isoliert aus der Wespe *Bracon hebetor,* das Insektentoxizität zeigt, ein Molekulargewicht von mehr als 70 kDa besitzt und die Sequenz von SEQ ID No. 5 besitzt.

4. Polypeptid, das Insektentoxizität zeigt, ein Molekulargewicht von mehr als 70 kDa besitzt und zu mindenstens 80% zu dem in Anspruch 3 definierten Polypeptid homolog ist.

5. Nukleinsäure, die ein Polypeptid nach einem der Ansprüche 1 bis 4 codiert.

6. Nukleinsäure nach Anspruch 5, die DNA ist.

7. Vektor, umfassend die Nukleinsäure nach Anspruch 5.

8. Baculovirus, dessen Genom eine Nukleinsäure nach Anspruch 5 umfaßt.

9. Verfahren zur Bekämpfung von Insekten, wobei man die Insekten einer insektenbekämpfenden Mengen eines Polypeptids nach einem der Ansprüche 1 bis 4 exponiert.

10. Verfahren zur Bekämpfung von Insekten, wobei man die Insekten mit einem in Anspruch 8 definierten Baculovirus infiziert.

## Revendications

1. Un polypeptide isolé de la guêpe Bracon Hebetor qui présente une toxicité envers les insectes, a un poids moléculaire supérieur à 70 kDa et comprend la séquence SEQ ID N° 1, SEQ ID N° 2 ou SEQ ID N° 3.

2. Un polypeptide qui présente une toxicité envers les insectes, a un poids moléculaire supérieur à 70 kDa et est au moins homologue à 80% à un des polypeptides définis à la revendication 1.

3. Un polypeptide désigné Brh-1 isolé de la guêpe Bracon hebetor qui présente une toxicité envers les insectes, a un poids moléculaire supérieur à 70 kDa et a la séquence SEQ ID N° 5.

4. Un polypeptide qui présente une toxicité envers les insectes, a un poids moléculaire supérieur à 70 kDa et est au moins homologue à 80% au polypeptide défini à la revendication 3.

5. Un acide nucléique codant un polypeptide selon l'une quelconque des revendications 1 à 4.

6. Un acide nucléique selon la revendication 5 qui est le DNA.

7. Un vecteur comprenant l'acide nucléique de la revendication 5.

8. Un baculovirus dont le génome comprend un acide nucléique de la revendication 5.

9. Une méthode de lutte contre les insectes comprenant l'exposition des insectes à une quantité luttant contre les insectes d'un polypeptide selon l'une quelconque des revendications 1 à 4.

10. Une méthode de lutte contre les insectes comprenant l'infection des insectes avec un baculovirus défini à la revendication 8.


### SEQUENCE LISTING

(1) GENERAL INFORMATION:
   (i) APPLICANT:Quistad, Gary B.
      Leisy, Douglas J.
   (ii) TITLE OF INVENTION: Insecticidal Toxins fro the Parasitic Wasp Bracon Hebetor
   (iii) NUMBER OF SEQUENCES: 13
   (iv) CORRESPONDENCE ADDRESS:
      (A) ADDRESSEE: Sandoz Agro Inc, Patent Dept.
      (B) STREET: 975 California Ave.
      (C) CITY: Palo Alto
      (D) STATE: CA
      (E) COUNTRY: USA
      (F) ZIP: 94304-1104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Giesser, Joanne M.
      (B) REGISTRATION NUMBER: 32,838
      (C) REFERENCE/DOCKET NUMBER: Z-703
   (ix) TELECOMMUNICATION INFORMATION:
      (A) TELEPHONE: (415) 354-3588
      (B) TELEFAX: (415) 857-1125
(2) INFORMATION FOR SEQ ID NO:1:
   (i) SEQUENCE CHARACTERISTICS:
      (A) LENGTH: 45 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (xi) SEQUENCE DESCRIPTION: SEQ ID NO:1:
   Xaa Thr Asp Phe Tyr Tyr Thr Asp Val Ile Ala Asp Gln Asp Phe Leu
   1               5                   10                  15
   Leu Lys Gln Lys Lys Val Phe Gln Leu Leu Tyr His Val Ser Gln Pro
               20                  25                  30
   Xaa Ile Ser Asn Xaa Xaa Xaa Phe Gln Xaa Xaa Leu Lys
           35                  40                  45
(2) INFORMATION FOR SEQ ID NO:2:
   (i) SEQUENCE CHARACTERISTICS:
      (A) LENGTH: 28 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (xi) SEQUENCE DESCRIPTION: SEQ ID NO:2:
   Xaa His Val Gln Thr Tyr Thr Ala Asp Met Asp Phe Lys His Lys Gln
   1               5                   10                  15
   Lys Lys Ile Tyr His Leu Phe Xaa Xaa Xaa Xaa Gln
               20                  25
(2) INFORMATION FOR SEQ ID NO:3:
   (i) SEQUENCE CHARACTERISTICS:
      (A) LENGTH: 41 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (ix) FEATURE:
      (A) NAME/KEY: Modified-site
      (B) LOCATION: 25..26
      (D) OTHER INFORMATION: /label= AA25
         /note= "Amino acid #25 may be I or Q."
   (xi) SEQUENCE DESCRIPTION: SEQ ID NO:3:
   Xaa Leu Phe Asp Phe Ile Val His Ala Lys Asp Ile Leu Gly Gly Ile
   1               5                   10                  15
   Asp Asn Leu Ala Lys Gly Ile Xaa Xaa Ala Ile Asn Lys Val Xaa Xaa
               20                  25                  30
   Val Ile Xaa Lys Val Gln Xaa Gln Ala
           35                  40
(2) INFORMATION FOR SEQ ID NO:4:
   (I) SEQUENCE CAARACTERISTICS:
      (A) LENGTH: 2387 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 85..2121
   (xi) SEQUENCE DESCRIPTION: SEQ ID NO:4:
   GCAACACAAG TGTTACTTCG TTTGCCACTT CACTGTTGAA GAAAAATAAA AAATACATTT      60
   TGATTATCAC TTGAATAATC TATA ATG CTG AAG AAG GTC TTT CTT TTG GCC       111
                              Met Leu Lys Lys Val Phe Leu Leu Ala
                               1               5
   TCT TTG GCT ATT ATC GTG ATA AAA GCT GAT ACC GAT TTT TAT TAT ACA      159
   Ser Leu Ala Ile Ile Val Ile Lys Ala Asp Thr Asp Phe Tyr Tyr Thr
    10                  15                  20                  25
   GAT GTG ATA GCT GAT CAA GAT TTC CTT TTA AAG CAA AAG AAA GTT TTT      207
   Asp Val Ile Ala Asp Gln Asp Phe Leu Leu Lys Gln Lys Lys Val Phe
                    30                  35                  40
   CAA TTG TTG TAT CAT GTT TCA CAA CCA GAC ATC TCA AAT CCC GAG CTT      255
   Gln Leu Leu Tyr His Val Ser Gln Pro Asp Ile Ser Asn Pro Glu Leu
                45                  50                  55
   TTC CAG GAG GGA TTG GCT TAT GAC ATT GGA GCC AAT ATT GAT TCC TAT      303
   Phe Gln Glu Gly Leu Ala Tyr Asp Ile Gly Ala Asn Ile Asp Ser Tyr
            60                  65                  70
   TCT AAT AAG GAT GCA GTG AAT CAC TTC CTC GAG CTA TAC AAA TTC GGA      351
   Ser Asn Lys Asp Ala Val Asn His Phe Leu Glu Leu Tyr Lys Phe Gly
        75                  80                  85
   TTC CTT CCA AGA GGT GCA ATC TAC TGG GTC TAT TAT CCT AAA CTC TTG      399
   Phe Leu Pro Arg Gly Ala Ile Tyr Trp Val Tyr Tyr Pro Lys Leu Leu
    90                  95                 100                 105
   GAC GAG ACT AAA GCC TTG TTC AAA TTG TTC TAC TAT GCC AAG GAC TTT      447
   Asp Glu Thr Lys Ala Leu Phe Lys Leu Phe Tyr Tyr Ala Lys Asp Phe
                   110                 115                 120
   GAT ACT TTC TAT AAA ACT GCC CTT TGG GCG AGA AAT CGT TTG AAC GAA      495
   Asp Thr Phe Tyr Lys Thr Ala Leu Trp Ala Arg Asn Arg Leu Asn Glu
               125                 130                 135
   GGT GAA TTC ATA TGT GCC TTC TAT GAA GCT GTC ATC CGG CGT CCC GAC      543
   Gly Glu Phe Ile Cys Ala Phe Tyr Glu Ala Val Ile Arg Arg Pro Asp
           140                 145                 150
   ACA GAG TAT CTC CAG TTA CCA CCG CCT TAT GAG TTA TAT CCC TAT GCG      591
   Thr Glu Tyr Leu Gln Leu Pro Pro Pro Tyr Glu Leu Tyr Pro Tyr Ala
       155                 160                 165
   TTC TTC AAC AGT GAG GTA ATC GAG GCT GCA AAA AAT GCC AAA TTG TAC      639
   Phe Phe Asn Ser Glu Val Ile Glu Ala Ala Lys Asn Ala Lys Leu Tyr
   170                 175                 180                 185
   AAT AAG CTT GTT GAA GGA AAT TCC TAC ATT ATC TAT GTC AAT TAC TCC      687
   Asn Lys Leu Val Glu Gly Asn Ser Tyr Ile Ile Tyr Val Asn Tyr Ser
                   190                 195                 200
   GGC TGG TAC TTG AAT CGA GCT TAT GAT ACA GAG ATG AGA GTC AAC TAT      735
   Gly Trp Tyr Leu Asn Arg Ala Tyr Asp Thr Glu Met Arg Val Asn Tyr
               205                 210                 215
   TTC CTC GAA GAT ATC GGT TTA AAC ACC TTC TAC TTT TTC TAC CGC ATG      783
   Phe Leu Glu Asp Ile Gly Leu Asn Thr Phe Tyr Phe Phe Tyr Arg Met
           220                 225                 230
   GAT AAT CCA TTT TGG TTG AGC AGT GAG GAA TTT GGT TTG CAG AAA AAT      831
   Asp Asn Pro Phe Trp Leu Ser Ser Glu Glu Phe Gly Leu Gln Lys Asn
       235                 240                 245
   TTG CGT GGT GAG GAA TTT CTC TAT GTT CAC AAA ACA CTC TTG AAT CGT      879
   Leu Arg Gly Glu Glu Phe Leu Tyr Val His Lys Thr Leu Leu Asn Arg
   250                 255                 260                 265
   TAC AAT TTG GAA AGA TTG GCA AAT GGC TTG GAG AAA ATT GAA GAA TTC      927
   Tyr Asn Leu Glu Arg Leu Ala Asn Gly Leu Glu Lys Ile Glu Glu Phe
                   270                 275                 280
   CTT TGG GAG GGA GAA TTT TAT CCA GGC TAT TAT CCA ACT ATG GTC TAT      975
   Leu Trp Glu Gly Glu Phe Tyr Pro Gly Tyr Tyr Pro Thr Met Val Tyr
               285                 290                 295
   GGC AAT GGG CTG GCT TAT CCT CAG CGT CCA GGA ATG AGT AGG ATT CCT     1023
   Gly Asn Gly Leu Ala Tyr Pro Gln Arg Pro Gly Met Ser Arg Ile Pro
           300                 305                 310
   CCA TAT AAG TAT CAT TAT TTA CGG TAT ATC CAC GAT ATC GAA GAT AGA     1071
   Pro Tyr Lys Tyr His Tyr Leu Arg Tyr Ile His Asp Ile Glu Asp Arg
       315                 320                 325
   ATT TCA ACA GCC ATT GAC TTG GGC TAT ATA ATC GAC AGC GAT GGT AGT     1119
   Ile Ser Thr Ala Ile Asp Leu Gly Tyr Ile Ile Asp Ser Asp Gly Ser
   330                 335                 340                 345
   CAC CAC AAC ATC TCA AGT CCC GAA GGA CTT AAC CTC TTA GGT AAC ATC     1167
   His His Asn Ile Ser Ser Pro Glu Gly Leu Asn Leu Leu Gly Asn Ile
                   350                 355                 360
   ATC GAG GGT AAT GAA GAT AGT TGC AAT AAA AAC TTT TAT CAC AGC CTC     1215
   Ile Glu Gly Asn Glu Asp Ser Cys Asn Lys Asn Phe Tyr His Ser Leu
               365                 370                 375
   GAT TGG TAT GGT AGA AAG GTT CTT GGT TTC AAT CTC GAG CCC AAG ACT     1263
   Asp Trp Tyr Gly Arg Lys Val Leu Gly Phe Asn Leu Glu Pro Lys Thr
           380                 385                 390
   CCC TAT CAA GTT ATT CCA AGT GCA CTA GAG TCA TTT TCA ACT TGC ATG     1311
   Pro Tyr Gln Val Ile Pro Ser Ala Leu Glu Ser Phe Ser Thr Cys Met
       395                 400                 405
   AGA GAT CCG GCT TTC TAT CGT CTC TAC AAT AGA TAT CTG TCA TAC TGG     1359
   Arg Asp Pro Ala Phe Tyr Arg Leu Tyr Asn Arg Tyr Leu Ser Tyr Trp
   410                 415                 420                 425
   TAC AGA TTC AAA GAA ACC TTG AAG CCA TAT TCT AAG AAT GAA ATA GTC     1407
   Tyr Arg Phe Lys Glu Thr Leu Lys Pro Tyr Ser Lys Asn Glu Ile Val
                   430                 435                 440
   TTC TCT GAT TTG AAA TTT GAA TCA ATT GCT GTT GAT AAA TTG ATC ACA     1455
   Phe Ser Asp Leu Lys Phe Glu Ser Ile Ala Val Asp Lys Leu Ile Thr
               445                 450                 455
   TAT TTT GAT TAT TTT GAT TCA ACA ATT AGC AAT GGT CTA CCA ATT ACA     1503
   Tyr Phe Asp Tyr Phe Asp Ser Thr Ile Ser Asn Gly Leu Pro Ile Thr
           460                 465                 470
   AGT AAA CAA GAT GCT GAT AAT TTA ATG ATC AAA GTT CGC CAG AGT CGT     1551
   Ser Lys Gln Asp Ala Asp Asn Leu Met Ile Lys Val Arg Gln Ser Arg
       475                 480                 485
   TTA AAT AAT AAA CAC TTT ACC GTA CAT TTC GCC CTA AAT TCC GAT AAA     1599
   Leu Asn Asn Lys His Phe Thr Val His Phe Ala Leu Asn Ser Asp Lys
   490                 495                 500                 505
   GCA CAA AAA GTT GCC ATT CAG CTG TTT CTT GGA CCC AAA TAT GAT GCA      1647
   Ala Gln Lys Val Ala Ile Gln Leu Phe Leu Gly Pro Lys Tyr Asp Ala
                   510                 515                 520
   CTT GGT AAT TTA TTG GAC TTT TCC GAG AGT TAC AAA GAC TTT TAT GAG      1695
   Leu Gly Asn Leu Leu Asp Phe Ser Glu Ser Tyr Lys Asp Phe Tyr Glu
               525                 530                 535
   ATT GAC TAC TGG ATT ACT GAT GTG AAT GCT GGC TTG AAT AAA CTT GAA      1743
   Ile Asp Tyr Trp Ile Thr Asp Val Asn Ala Gly Leu Asn Lys Leu Glu
           540                 545                 550
   CGT ACC AGT CAC GAC TTT ATC TTT TTG ATG GCC GAC CGA GAT CCA AGT      1791
   Arg Thr Ser His Asp Phe Ile Phe Leu Met Ala Asp Arg Asp Pro Ser
       555                 560                 565
   GAA ATT TTA TAC AAA AGA GTT TTA AAG GCC CTT GAT GGA AGT GAA AAG      1839
   Glu Ile Leu Tyr Lys Arg Val Leu Lys Ala Leu Asp Gly Ser Glu Lys
   570                 575                 580                 585
   TTC ATG TAC AAA AAG AAT TTG TAT GGC ATT CCG GAA CGT TTA CTT CTA      1887
   Phe Met Tyr Lys Lys Asn Leu Tyr Gly Ile Pro Glu Arg Leu Leu Leu
                   590                 595                 600
   CCA AAA GGT AAA CGT GCC GGT AGT ATT TTC CAA CTG TTT GCC TAT GTA      1935
   Pro Lys Gly Lys Arg Ala Gly Ser Ile Phe Gln Leu Phe Ala Tyr Val
               605                 610                 615
   AGC CCA GTT ACC CAG CCA GTC ACC TAC AAA TCA CGA GTA TTT GGA TCT      1983
   Ser Pro Val Thr Gln Pro Val Thr Tyr Lys Ser Arg Val Phe Gly Ser
           620                 625                 630
   TAT CAA TAC TAC ATG AAA CCA GGT GGT TTT CCA CTG GAC AGG CCA ATC      2031
   Tyr Gln Tyr Tyr Met Lys Pro Gly Gly Phe Pro Leu Asp Arg Pro Ile
       635                 640                 645
   TAC TAT CCC CAT TTC CAA GGG CCC AAT ATG TTC TTC AAA GAT ATT ACG      2079
   Tyr Tyr Pro His Phe Gln Gly Pro Asn Met Phe Phe Lys Asp Ile Thr
   650                 655                 660                 665
   ATT TAC CAC AAG ACT GAT GTG GAT CCT AAT GCT ACT ACC TAATTCCAAT       2128
   Ile Tyr His Lys Thr Asp Val Asp Pro Asn Ala Thr Thr
                   670                 675
   TTTTTTACTC TATTTTCATT TGAGATTCTT ATCAAATTCA ATGTTTGTTT GTTAATATTG    2188
   TCTTTGTAGA GCTTAGAATG TTAGATTGAA AATGTTTATT TCCATGACAA TTTATTATTT    2248
   GTTATTGATA TTATCAATGA ATTCTCTGTC AGTCAACCTC AGAGAATATA AAATTTTATT    2308
   ACAAAAATGT CGTATTGAGC ATAAATTCAT TATTTGGGAA AAATTTTCAA ATAAAAAGCA    2368
   TATTTTCCAA CAAAAAAAA                                                 2387
(2) INFORMATION FOR SEQ ID NO:5:
   (i) SEQUENCE CHARACTERISTICS:
      (A) LENGTH: 678 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Leu Lys Lys Val Phe Leu Leu Ala Ser Leu Ala Ile Ile Val Ile
    1               5                  10                  15
   Lys Ala Asp Thr Asp Phe Tyr Tyr Thr Asp Val Ile Ala Asp Gln Asp
               20                  25                  30
   Phe Leu Leu Lys Gln Lys Lys Val Phe Gln Leu Leu Tyr His Val Ser
           35                  40                  45
   Gln Pro Asp Ile Ser Asn Pro Glu Leu Phe Gln Glu Gly Leu Ala Tyr
       50                  55                  60
   Asp Ile Gly Ala Asn Ile Asp Ser Tyr Ser Asn Lys Asp Ala Val Asn
   65                  70                  75                  80
   His Phe Leu Glu Leu Tyr Lys Phe Gly Phe Leu Pro Arg Gly Ala Ile
                   85                  90                  95
   Tyr Trp Val Tyr Tyr Pro Lys Leu Leu Asp Glu Thr Lys Ala Leu Phe
               100                 105                 110
   Lys Leu Phe Tyr Tyr Ala Lys Asp Phe Asp Thr Phe Tyr Lys Thr Ala
           115                 120                 125
   Leu Trp Ala Arg Asn Arg Leu Asn Glu Gly Glu Phe Ile Cys Ala Phe
       130                 135                 140
   Tyr Glu Ala Val Ile Arg Arg Pro Asp Thr Glu Tyr Leu Gln Leu Pro
   145                 150                 155                 160
   Pro Pro Tyr Glu Leu Tyr Pro Tyr Ala Phe Phe Asn Ser Glu Val Ile
                   165                 170                 175
   Glu Ala Ala Lys Asn Ala Lys Leu Tyr Asn Lys Leu Val Glu Gly Asn
               180                 185                 190
   Ser Tyr Ile Ile Tyr Val Asn Tyr Ser Gly Trp Tyr Leu Asn Arg Ala
           195                 200                 205
   Tyr Asp Thr Glu Met Arg Val Asn Tyr Phe Leu Glu Asp Ile Gly Leu
       210                 215                 220
   Asn Thr Phe Tyr Phe Phe Tyr Arg Met Asp Asn Pro Phe Trp Leu Ser
   225                 230                 235                 240
   Ser Glu Glu Phe Gly Leu Gln Lys Asn Leu Arg Gly Glu Glu Phe Leu
                   245                 250                 255
   Tyr Val His Lys Thr Leu Leu Asn Arg Tyr Asn Leu Glu Arg Leu Ala
               260                 265                 270
   Asn Gly Leu Glu Lys Ile Glu Glu Phe Leu Trp Glu Gly Glu Phe Tyr
           275                 280                 285
   Pro Gly Tyr Tyr Pro Thr Met Val Tyr Gly Asn Gly Leu Ala Tyr Pro
       290                 295                 300
   Gln Arg Pro Gly Met Ser Arg Ile Pro Pro Tyr Lys Tyr His Tyr Leu
   305                 310                 315                 320
   Arg Tyr Ile His Asp Ile Glu Asp Arg Ile Ser Thr Ala Ile Asp Leu
                   325                 330                 335
   Gly Tyr Ile Ile Asp Ser Asp Gly Ser His His Asn Ile Ser Ser Pro
   340 345 350
   Glu Gly Leu Asn Leu Leu Gly Asn Ile Ile Glu Gly Asn Glu Asp Ser
   355 360 365
   Cys Asn Lys Asn Phe Tyr His Ser Leu Asp Trp Tyr Gly Arg Lys Val
   370 375 380
   Leu Gly Phe Asn Leu Glu Pro Lys Thr Pro Tyr Gln Val Ile Pro Ser
   385 390 395 400
   Ala Leu Glu Ser Phe Ser Thr Cys Met Arg Asp Pro Ala Phe Tyr Arg
   405 410 415
   Leu Tyr Asn Arg Tyr Leu Ser Tyr Trp Tyr Arg Phe Lys Glu Thr Leu
   420 425 430
   Lys Pro Tyr Ser Lys Asn Glu Ile Val Phe Ser Asp Leu Lys Phe Glu
   435 440 445
   Ser Ile Ala Val Asp Lys Leu Ile Thr Tyr Phe Asp Tyr Phe Asp Ser
   450 455 460
   Thr Ile Ser Asn Gly Leu Pro Ile Thr Ser Lys Gln Asp Ala Asp Asn
   465 470 475 480
   Leu Met Ile Lys Val Arg Gln Ser Arg Leu Asn Asn Lys His Phe Thr
   485 490 495
   Val His Phe Ala Leu Asn Ser Asp Lys Ala Gln Lys Val Ala Ile Gln
   500 505 510
   Leu Phe Leu Gly Pro Lys Tyr Asp Ala Leu Gly Asn Leu Leu Asp Phe
   515 520 525
   Ser Glu Ser Tyr Lys Asp Phe Tyr Glu Ile Asp Tyr Trp Ile Thr Asp
   530 535 540
   Val Asn Ala Gly Leu Asn Lys Leu Glu Arg Thr Ser His Asp Phe Ile
   545 550 555 560
   Phe Leu Met Ala Asp Arg Asp Pro Ser Glu Ile Leu Tyr Lys Arg Val
   565 570 575
   Leu Lys Ala Leu Asp Gly Ser Glu Lys Phe Met Tyr Lys Lys Asn Leu
   580 585 590
   Tyr Gly Ile Pro Glu Arg Leu Leu Leu Pro Lys Gly Lys Arg Ala Gly
   595 600 605
   Ser Ile Phe Gln Leu Phe Ala Tyr Val Ser Pro Val Thr Gln Pro Val
   610 615 620
   Thr Tyr Lys Ser Arg Val Phe Gly Ser Tyr Gln Tyr Tyr Met Lys Pro
   625 630 635 640
   Gly Gly Phe Pro Leu Asp Arg Pro Ile Tyr Tyr Pro His Phe Gln Gly
   645 650 655
   Pro Asn Met Phe Phe Lys Asp Ile Thr Ile Tyr His Lys Thr Asp Val
   660 665 670
   Asp Pro Asn Ala Thr Thr
   675
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:6:
   ARYTGRAANA CYTTYTTYTG 20
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:7:
   GAYTTYTAYT AYACNGAYGT 20
(2) INFORMATION FOR SEQ ID NO:8:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:8:
   GGAAATCTTG ATCAGCTATC AC 22
(2) INFORMATION FOR SEQ ID NO:9:
   (i) SEQUENCE CHARACTERISTICS:
      (A) LENGTH: 31 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (xi) SEQUENCE DESCRIPTION: SEQ ID NO:9:
   Thr Asp Phe Tyr Tyr Thr Asp Val Ile Ala Asp Gln Asp Phe Leu Leu
   1               5                   10                  15
   Lys Gln Lys Lys Val Phe Gln Leu Leu Tyr His Val Ser Gln Pro
               20                  25                  30
(2) INFORMATION FOR SEQ ID NO:10:
   (i) SEQUENCE CHARACTERISTICS:
      (A) LENGTH: 93 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:10:
   ACNGAYTTYT AYTAYACNGA YGTNATHGCN GAYCARGAYT TYYTNYTNAA RCARAARAAR 60
   GTNTTYCARY TNYTNTAYCA YGTNWSNCAR CCN 93
(2) INFORMATION FOR SEQ ID NO:11:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:11:
   GATTTTTATT ATACTGATGT GATAGCTGAT CAAGATTTCC TTTTAAAGCA AAAGAAGGTT 60
   TTTCAATT 68
(2) INFORMATION FOR SEQ ID NO:12:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:12:
   GACTTTTATT ACACTGACGT GATAGCTGAT CAAGATTTCC TTTTAAAGCA AAAAAAGGTA 60
   TTTCAACT 68
(2) INFORMATION FOR SEQ ID NO:13:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:13:
   GATTTTTATT ATACTGATGT GATAGCTGAT CAAGATTTCC TTTTAAAGCA AAAGAAGGTA    60
   TTTCAACT                                                             68